(19) Europäisches Patentamt
European Patent Office
Office européen des brevets

(11) EP 0 381 823 B1

(12) EUROPEAN PATENT SPECIFICATION

(45) Date of publication and mention of the grant of the patent: **11.09.1996 Bulletin 1996/37**

(51) Int. Cl.[6]: **A61K 31/557**, A61K 31/59, A61K 31/07, A61K 31/355, A61K 31/20

(21) Application number: **89121224.3**

(22) Date of filing: **16.11.1989**

# (54) Cytotropic heterogenous molecular lipids (CHML) and process for preparing the same

Zytotropische heterogene molekulare Lipide und Verfahren für ihre Zubereitung

Lipides moléculaires hétérogènes cytotropiques et leur procédé de préparation

(84) Designated Contracting States:
**CH DE FR LI**

(30) Priority: **16.11.1988 CN 88108000**

(43) Date of publication of application:
**16.08.1990 Bulletin 1990/33**

(73) Proprietor: **Xu, Zheng**
**Qing-Pu Town Shanghai (CN)**

(72) Inventor: **Xu, Zheng**
**Qing-Pu Town Shanghai (CN)**

(74) Representative: **von Füner, Alexander, Dr. et al**
**Patentanwälte v. Füner, Ebbinghaus, Finck**
**Mariahilfplatz 2 & 3**
**81541 München (DE)**

(56) References cited:
- **"The Merck Index", 11th edition, 1989, pages 867,1079,1080,1107,1252,1383,1386, 1576,1578,1579, Merck & Co., Inc., Rayway, NY, US**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Rank Xerox (UK) Business Services
2.13.4/3.4

## Description

This invention relates to cytotropic heterogenous molecular lipids.

In the early stage of 60's, British scientist Bangham and others found that phosphatide may form small multilayer capsules while dispersing in water, and discovered that each layer of bimolecular lipids in multilayer cyst is separated by water, the thickness of each layer is about 40 Å. This kinds of micro particles of the capsules, having structure similar to biomembrane, are so named liposome.

Owing to that it comprises a hydrophilic and/or lipophilic "small room". These "small rooms" of liposome may envelop those molecules and ions which are soluble in water or lipoid. These specific characteristics makes the liposome can be widely used as carrier, especially for pharmaceutical. Further, liposome may alter the mechanism of pharmaceutical metabolism and selectively transport the pharmaceutical to the "target", then release the pharmaceutical at the proper part of tissues to be cured. Thus, the toxicity to the normal cells can be reduced and the curative effect to attack deleterious cell can be greatly enhanced. In addition, there is another specific property that the enveloped pharmaceutical by liposome may be slowly released in local site whether entering into blood circulation or combining with cells and tissues, even entering into cells through pinocytosis. With slow release mechanism of liposome, the half-life of the effective pharmaceutical is prolonged and the therapeutic effect is obviously improved,. Furthermore, liposome is made from natural phosphatides and cholesterols, havin low toxicity, free from immunogen with suitable bio-compatibility and bio-degradable properties. Recently, liposome engineering has been made progress and thus the application of liposome for a carrier of pharmaceutical has been possibly promoted.

In 1971, British scientist Ryman and others suggested that liposome could be used as carrier for enzymes or pharmaceutical. Based on the research of biomembrane theory, Ryman started to envelop enzymes or pharmaceutical into liposome, in order to enable for the active substances or pharmaceutical to avoid destruction in blood circulation and to selectively attack deleterious cells of the target tissue.

Chinese Canadian scientist Chang Ming-Su had researched these carriers, it is enveloped with pharmaceutical in therapic usage. In 1985, famous Chinese professor Gu Xueqiu got the highest honor at the Fourth International Cancer of Lungs Conference upon reporting some successful cases in 1980's in two kinds of polyphase liposome as anti-cancer pharmaceutical carrier in combination with other chemical drugs to combat cancer.

Polyphase liposome is a new form of anti-cancer pharmaceutical carrier of polyphase-dispersing system. Enveloping some anti-cancer pharmaceutical into the carrier and entering into blood circulation of the patient intravenously injected, the polyphase liposome can exactly hit the target cancer tissue as a so-called "ultra-micro missile".

The polyphase liposome is of a milky-white emulsion. Several kinds of emulsive intravenous injection developed are composed of ultra-micro particles of drug carrier, having lympha system orientation, some of them penetrate the lysosome through phagocytosis of the phagocyte of dicty endothelial system, then the pharmaceutical is released upon digesting. Others release the pharmaceutical upon digesting by means of fusion function, namely, due to its material of membrane is similar to that of the cell membrane, the polyphase liposome fused into the cell, thus maintaining rather high concentration significantly reduces the toxicity and attack of anti-cancer pharmaceutical will be enhanced while the toxicity to the normal cells being reduced.

In general, liposome may be deemed to be an artificial membrane of cell, having an enclosed spheroidal structure with the diameter about 300-2000Å and maximum diameter about 5μ, keeping drug well in the small room to function. But, in generally speaking, the particle sizes of such liposome having relatively large diameter could hardly penetrate into the cytoplasm directly, it is necessary for liposome to be phagocytized by the cell.

Up to the present, there have been in the prior art liposomes which mostly comprise phosphatide as a skeleton material and additives. Phosphatide is an amphoteric compound possessing hydrophilic and lipophilic radicals, including natural phosphatide (lecithin and soy-bean lecithin) and synthetic phosphatide such as phosphatidyl choline (dipalmitol phosphatidyl choline, distearyl phosphatidyl choline). These phosphatides provide two hydrophilic chains. They form liposome of bi-mocular layer in water, no matter how the structure of the hydrophilic radical is. The additives used in the prior art are, for example, cholesterol, octadecamine and phosphatidate, etc. Though cholesterol is useful for regulating the flowability and permeability of bi-molecular layer but cholesterol is no good for human being. Octadecamine and phosphatidate may be used to alter the surface electrical charges of liposome. The components of polyphase liposome may be phosphatide, oleic acid, cholesterol and nonionic surfactants such as PVP (polyvinylpyrrolidone).

Many research works and applications of liposome as drug carriers and used as models of bio-membrane and methods of its preparation ore developed to a certain extent. The polyphase liposome is formed after the phosphatides contact with water, is due to the action of its polar group and hydrophobic group and arranged in the form of poly bimolecular layer of spherical structure in closed type. The water layer is laid between the layers of bimolecular as the water-soluble drugs are enveloped into it, while the liposoluble pharmaceuticals are enveloped in the bimolecular layers. Many factors as surface characteristics, particle sizes, differences in forms, surface electrical charges of the liposome can effect the stability in vivo and the percentages of enveloped pharmaceutical. The factors are dependent upon the components of phosphatides and methods of preparation.

Chemical properties of phosphatide with unsaturated fatty acid chain, such as of lecithin and soy bean lecithin are sometimes not sufficiently stable. Phosphatide is susceptible to oxidation and hydrolysis. Thus, peroxides, propane dial and lysophosphatide are produced. The oxidation of lecithin will subject the membrane formed to decrease flowability and to increase stability and negative electrical charge condition. Thus leakage of drugs will be promoted so that retaining of drugs will become less and the liposome will be easily aggregated and precipitated. Thereby toxicity is produced. Therefore, it has been suggested that lecithin as a membrane should have high purity and an oxidation index of less than 0.2.

In preparation of liposome it is a difficult problem in the envelopment quantity of drug which has not yet been solved or ever since. For example, where the liposolubility and aqueous solubility of pharmaceutical both are low the envelopment quantity of the drug will be less. And where the molecule of the drug is small and easy to subject to percolation, the envelopment quantity of the drug will be weaker.

In the prior art, generally speaking, it has been known that liposome or polyphase liposome possesses advantages as a carrier of pharmaceutical. For example, they may enter into cells as a carrier of pharmaceutical for the pharmaceutical to keep therapeutic effect for prolonged duration owing to that the pharmaceutical enveloped by the carrier will be avoid destruction in blood circulation. During preparation they can envelop the pharmaceutical which may be hydrophilic or lipophilic. They may be formulated as "water in oil" or "oil in water" type emulsions, so that they may be phagocytized by cells through phagocytosis when they touch the surface of the cells. Furthermore, due to their lympha system orientation and selectivity, the amount of their entering into the lamphoglandulae, which is full of dicty endothelium cells, will be large, etc. However, in the data reported in the prior art there are some shortcomings at the present time in liposome or polyphase liposome, such as, their particle sizes formed are relatively large, which is about ten times bigger than the CHML of present invention such that they might cause systemic capillary circulation obstacle when it enters the vein. They should be phagocytized by cells to enter them, thus there must be an energy consumption in the cells. Their idiosyncratic phagocytosis by deleterious cells system in cancer cell is not so strong that they shall be often phagocytized by normal cells, causing the normal cells to be poisened and the bioeffect of the pharmaceutical to be reduced. Their promotion immunocyte of human being is seldom reported in the prior art. They could not envelop two or more phases of pharmaceutical in the same layer. They contain cholesterol and polyvinyl pyrrolidone (PVP) which are exert unhealthy effects to human being, etc.

In view of the foregoing, the main object of this invention is to provide lipids with very small size it is on the order of molecular dimension having active of orientation to penetrate into the membrane or wall of the cells, and having relatively high idiosyncratic absorbing capacity by target cells, so as to function as a "molecular missile" carrying only a small amount of pharmaceutical is necessary.

Another object of this invention is to provide such lipids on the order of molecular dimension comprising no phosphatide plus cholesterol system thus not having any disadvantageous factors from that system.

A further object of the invention is to provide such lipids on the order of molecular dimension, comprising no phosphatide plus cholesterol system, which itself have the effect of promotion for the immunity function of human being per se, and which itself have the effect of kill off the cells of therioma virus.

These objects were solved by a pharmaceutical composition which comprises:

0.5 - 2.0 wt. % squalene;
0.7 - 2.5 wt. % γ-linolenic acid;
1.0 - 4.0 wt. % linolenic acid;
14 - 28 wt. % oleic acid;
5 - 10 wt. % stearic acid;
6.5 - 12 wt. % palmitic acid;
3 - 7 wt. % eicosatetraenoic acid;
8 - 11 wt. % eicosapentaenoic acid;
2 - 4 wt. % eicosatrienoic acid;
10 - 15 wt. % docosahexenoic acid;
2 - 4 wt. % tetracosenoic acid;
4 - 6 wt. % docosenoic acid;
0.5 - 2 wt. % docosadienoic acid;
1 - 3 wt. % docosatrienoic acid;
3 - 7 wt. % docosatetraenoic acid;
5 - 10 wt. % docosapentaenoic acid;
10 - 20 wt. % palmitoleic acid;
3 - 7 wt. % Vitamin E;
0 - 0.5 wt. % Vitamin D; and
0 - 0.5 wt. % Vitamin A.

Other and further objects, features and advantages of the invention will appear more fully from the following description.

Upon investigation, it has been found that the molecule structure of liposome in the prior art can be described as a "stick", in which the handle is the hydrophilic radical of phosphatide in combination with cholesterol, while the straight rod portion of the stick is the place where the molecule of phosphatide and the like located. Phosphatide consists of phosphoric acid and fatty acid with the gathering of those sticklike molecules, the liposome of multilayer cyst layers, which is of hydrophilic and lipophilic, it appears to be irregular spherical particle under the microscope, the diameter of which is approximately 5μm.

Through a lot of experiments, "molecular lipids" have been successfully prepared by the inventor, which may be deemed to be a segment of the straight rod of the stick, namely, it comprises only the fatty acid part of the liposome getting rid of both handle and phosphoric acid part. Thus, the "molecular lipids" having significant therapeutic effects has been obtained, which need not to gather together to form relatively large "body" of particle multilayers, and each molecule of which is an element particle of the "lipids" with polar and non-polar groups to carry pharmaceutical. This is due to that the molecular lipids of the invention are of the following molecular formula (I) except squalene:

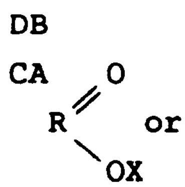

or

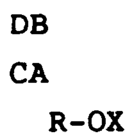

(I)

wherein C = carbon, DB = double bonds (0-8), CA = carbon atom, R = fundamental radical, O = oxygen, X = polarradical metal or non-metal ion, such as ions of Pt, Cu, Zn, Fe, Co, Mg, Ca, Na, H, and or any other positive ions, or negative ions of Cl, F, Br, I, N, Se and radicals of alcohol form, phenol form, ether form, aldehyde form, in keto-form and quinoid-form, or radicals of pyrimidine molecule and purine molecule or amino-acid molecule, glucose molecule. OX are hydrophilic radical and R is lipohilic radical.

Being a molecular carrier, in the said molecular structure there is a part CA which is able to bind the molecule of anti-cancer drugs. X is also a part where the anticancer drug molecule can be bound. While DB, CA, O and X are active parts to the membraneous structure of cancer cells.

The hydrophilic radical of the molecule of lipids comes into contact with the surface of outer membrane of the cell first while the molecule is self rotating, and then spirally turning for 180° so as for the hydrophobic radical of the molecule to enter into the membrane interval. Consequentially the whole molecule gets into the membrane interval. Afterwards, the hydrophobic radical of the molecule of lipids comes into contact with the inner surface of the membrane interval, spirally turning for 180° once more, and then the hydrophilic radical goes beyond the inner surface of the membrane interval and coming into cytoplasma of the cell. This is the mechanism for the molecular lipids of the invention to enter into the cell in turning 360°.

Most of the external lipids must be hydrolized into molecular lipids when it is to be absorbed by the biomembrane. The molecular lipids possess amphotericity of hydrophilic and lipophilic properties. The molecular lipids of the membrane of cells comprise various saturated fatty acids, unsaturated fatty acids, phosphoric acids, glycerine, cholesterol and liposoluble vitamines, etc. Separating the inner part of the cell from outside, these substances constitute the lipid osmotic pressure of the cell membranes. Under certain conditions, for example, under ultraviolet rays, the saturated and the unsaturate fatty acid, co-existing in the biomembrane, shall be interconvertable by means of dehydrogenation. The saturated fatty acid in biomembrane increases the viscosity and stability of the biomembrane to enhance relatively the capillary circulation, while the unsaturated fatty acid in biomembrane increases the fluidity and instability of bio-membrane to diminish relatively the capillary circulation. Conversely, when the saturated fatty acid in blood increases, the amount of blood in capillary circulation will be relatively diminished, and when the unsaturated fatty acid in blood increases, the amount of blood in capillary circulation will be relatively increased.

When the external lipids have just arrived the saturated concentration of lipids of cells in inside and outside dynamic balance, attractive force to the lipids will disappear from the inside so that the molecular lipids shall not be able to enter into the inside of the cells. The magnitude of the osmotic pressure of the external molecular lipids has concern with the hydrophilic-lipophilic balance (HLB) of the biomembrane molecular lipids and with the external molecular lipids itself. The osmotic ratio (osmotic coefficient) of the external molecular lipids is directly (positively) proportional to surface area, and inversely proportional to magnitude of the external molecular lipids. When normal human is in a hungry state, the osmotic pressure of lipids within and without the cell is in dynamic balance, attractive force to the lipids will disappear. So that the molecular lipids shall not be able to enter the cell. The infiltrate osmotic pressure in the state of dynamic balance is averagely about 0.127-0.318 atm. (96.52-241.47 mm Hg). The external factors such as physical,

chemical and biological factors shall first raise the lipid osmotic pressure owing to the change in osmotic pressure of the ions in the membrane, thus thinning out of the membrane structure with the traction. With the percolation of the cholesterol and the lipids, the biomembrane will be heavily damaged and the osmotic pressure of lipids will be diminished. Using molecular lipids which are originated from normal cells of animals and plants and are essential to human being, the present invention has achieved the goal to control the osmotic pressure in abnormal cells or cancer cells and to effectively inhibit the growth, propagation and mitosis of the abnormal cells and the cancer cells. Where it is carrying anti-cancer drugs, the molecular lipids provided by the present invention shall provide more strong synergetic effects.

For more than ten years researching and experimenting, the inventor has sucessfully discovered and prepared a composition of molecular lipids to achieve the objects put forth in the present invention, which is named by the inventor "CHML" in abbreviation of "Cytotropic Heterogenous Molecular Lipid".

C, cytotropic, means that the lipid has an affinity for cytological cells. Owing to that the substances of the lipid are extracted from biological cells, or, the biological cells comprise the substances of the lipid, the substances of the lipid are of affinity for the membrane structure of any living cell, especially for the membrane structure of abnormal cells. At the same time, the lipid possesses the effect on stimulation of immunocyte, particularly of B-immunocyte and phagocyte (of mono-nuclear phagocyte system) to produce immunity reaction. Thus, CHML shall possess the ability of activating immunocyte to serve the function of immunity.

H, heterogenous, means that the lipid has the properties in physics, chemistry and biology which are similar to those of biomembrane. Especially the lipid possesses the properties for idiosyncratic absorption to the abnormal membrane structure, while it is capable of compatibility with water solution of polar molecule or water-soluble drugs as well as with liposoluble substances. Sometimes even molecules of gases can be dissolved in the lipid. On the other hand, H stands for that the attractive forces between the atoms exerted by the atoms of the elements in the molecule structure of the lipid are not equal, since there is positive-negative weak electrode at the both ends of the molecule, thus the lipid per se is of heterogeneity.

M, molecular, means that the lipid is of molecular size, having average molecular weight of about 300 and dimension of about (20-30) x (8-10) x (4-6) Å (Fig. 1), being less than 1/10 of the size of liposome in the prior art. The lipid exhibits as a transparent clear liquor (it can be explained by Tyndall effect).

L, lipid, means that the molecular composition structure and therapeutic effects of the carrier provided by the invention are quite different from that of the liposome in the prior art, and are of novelty and unique.

The molecular structure of CHML is relatively small so that it can easily penetrate into the biomembrane of the target abnormal cells to enter into the target cells, while liposome or polyphase liposome enters into the cells mainly in dependance on phagocytosis of the cells.

Playing the significant role of a carrier as a "biomolecular missile", CHML of the invention is able to strongly kill the cancer cells as well. It has been discovered that the abnormal metabolism of the lipoid substances in the cancer cells. The experiments in vitro have shown that CHML is able to destroy the sarcoma cells of S180 within 50 minutes (Fig. 3).

As a molecular carrier, CHML gives full play to carrying not only the hydrophilic and lipophilic molecules of pharmaceutical but also the gas-carrying drugs to enter into the target cells. Simultaneously, CHML has the compatibility with more than two phases of drugs to carry into therapeutic effects, while liposome or polyphase liposome in the prior art is seldom reported in this respect.

CHML of this invention functions in promotion for immunity of the cells of human being, particularly for B-immunocyte. In the prior art there has been hardly reported in this regard.

CHML of the invention is substantially extracted from cells of natural organisms. In other words, the cells of natural organisms contain the components of CHML of this invention. CHML provides little harmful side effects to the human being, and possessing good room temperature stability to be kept in store for more than two years at atmospheric pressure without deterioration of its properties. CHML of this invention does not contain phosphatide and the additives such as PVP (polyvinyl pyrrolidone) and the like, while it tends to diminish the toxicity and side-effects of the drugs carried.

The CHML of the invention will be of great value to the wide application in medical science and other fields. Particularly, CHML of the invention has the function of anti-hypertension and of obvious inhibition on various viruses.

The CHML of this invention comprises the saturated fatty acids, unsaturated fatty acids and liposoluble vitamines which are listed in Table 1.

Table 1

(1) Palmitic acid
$CH_3(CH_2)_{14}COOH$
$C_{16}H_{32}O_2 = 256$

(2) Stearic acid
$CH_3(CH_2)_{16}COOH$
$C_{18}H_{36}O_2 = 284$

(3) Palmitoleic acid (mainly Δ9- hexadecenoic acid)
$CH_3(CH_2)_5CH = CH(CH_2)_7COOH$
$C_{16}H_{30}O_2 = 254$

(4) Oleic acid (mainly Δ-9 octadecaenoic acid)
$CH_3(CH_2)_7CH = CH(CH\ )_7COOH$
$C_{18}H_{34}O_2 = 282$

(5) linolenic acid (mainly Δ9, 12, 15 octadecatrienoic acid)

$CH_3(CH_2)$ CH = $CHCH_2CH$=$CHCH_2CH$ = $CH(CH_2)_7COOH$
$C_{18}H_{30}O_2$ = 278

(6) γ-linolenic acid (mainly Δ 6, 9, 12 octadecatrienoic acid)
$CH_3CH_2CH_2CH_2CH_2CH$ = $CHCH_2CH$ = $CHCH_2CH$ =$CH(CH_2)$ COOH
$C_{18}H_{30}O_2$= 278

(7) Eicosatetraenoic acid (mainly Δ5, 8, 11, 14 eicosatetraenoic acid)
$CH_3(CH_2)_4$ CH = $CHCH_2CH$ =$CHCH_2CH$ = $CHCH_2CH$ = $CH(CH_2)_3$ COOH
$C_{20}H_{32}O_2$ = 304

(8) Eicosapentaenoic acid (mainly EPA)
$CH_3CH_2CH$ = $CHCH_2CH$ = $CHCH_2CH$ =$CHCH_2CH$ = $CHCH_2CH$ = $CHCH_2$ $CH_2CH_2COOH$
$C_{20}H_{30}O_2$ = 302

(9) Docosahexenoic acid (mainly DHA)
$CH_3CH_2CH$ = $CHCH_2CH$ =$CHCH_2CH$ = $CHCH_2CH$ = $CHCH_2CH$ = $CHCH_2$ CH = $CHCH_2CH_2COOH$
$C_{22}H_{32}O_2$ = 328

(10) Eicosatrienoic acid (mainly Δ9, 14, 17-eicosatrienoic acid
Δ8, 11, 14-eicosatrienoic acid)
$CH_3(CH_2)_3CH$ = $CHCH_2CH$ =$CHCH_2CH$ = $CH(CH_2)_7COOH$
$CH_3(CH_2)_4CH$ = $CHCH_2CH$ = $CHCH_2CH$ = $CH(CH_2)_6COOH$
$C_{20}H_{34}O_2$= 306

(11) Docosadienoic acid (mainly Δ9.11-docosadienoic acid)
$CH_3(CH_2)_8CH$ = $CHCH_2CH$ = $CH(CH_2)_7COOH$
$C_{22}H_{40}O_2$ = 336

(12) Docosenoic acid (mainly Δ11-docosenoic acid)
$CH_3(CH_2)_9CH$ = $CH(CH_2)_9COOH$
$C_{22}H_{42}O_2$ = 338

(13) Docosatrienoic acid (mainly Δ9, 12, 15-docosatrienoic acid
Δ8, 11, 14-docosatrienoic acid)
$CH_3(CH_2)_5CH$ =$CHCH_2CH$ = $CHCH_2CH$ = $CH(CH_2)_7$ COOH
$CH_3(CH_2)_6CH$ = CHCH CH = CHCH CH =$CH(CH_2)_6COOH$
$C_{22}H_{38}O_2$ = 334

(14) Docosatetraenoic acid (mainly Δ7, 10, 13, 16-docosatetraenoic acid Δ6, 9, 12, 15-docosatetraenoic acid)

$CH_3(CH_2)_4CH = CHCH_2CH = CHCH_2CH = CHCH_2CH = CH(CH_2)_5COOH$

$CH_3(CH_2)_5CH = CHCH_2CH = CHCH_2CH = CHCH_2CH = CH(CH_2)_4COOH$

$C_{22}H_{36}O_2 = 332$

(15) Docosapentaenoic acid (mainly Δ 5, 8, 11, 14, 17-docosapentaenoic acid)

$CH_3(CH_2)_3CH = CHCH_2CH = CHCH_2CH = CHCH_2CH = CHCH_2CH = CH(CH_2)_3COOH$

$C_{22}H_{34}O_2 = 330$

(16) Tetracosenoic acid (mainly Δ12-tetracosenoic acid)

$CH_3(CH_2)_{10}CH = CH(CH_2)_{10}COOH$

$C_{24}H_{46}O_2 = 366$

(17) Squalene (mainly Δ2, 6, 10, 14, 19, 22 triacohexaenoic acid)

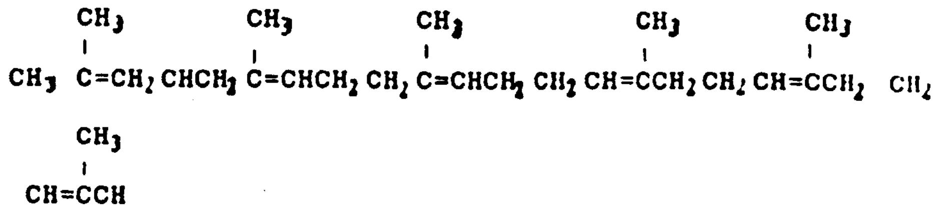

$C_{30}H_{50}$ = 410

(18) Vitamin A

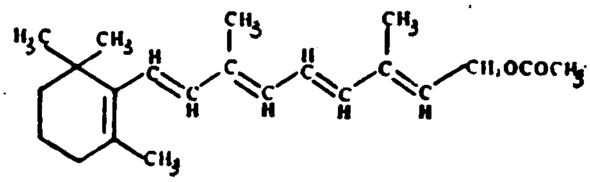

$C_{22}H_{32}O_2$ = 328

(19) Vitamin D

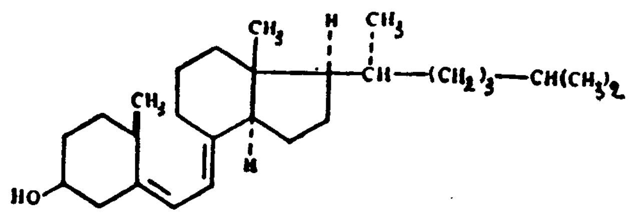

$C_{27}H_{44}O$ = 384

(20) Vitamin E

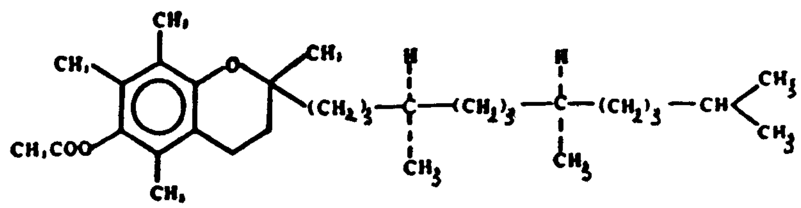

$C_{31}H_{52}O_3$ = 472

The composition of the CHML is substantially extracted from the cells of animals and plants and may be obtained by any methods in laboratories and industries. No matter how they are available, the substances need to be determined by qualitative and quantitative analyses prior to use, to satisfy the requirements of the composition provided. There are proper methods for the composition of the molecular lipids to be determined in qualitative and quantitative analyses, such as the application of fatty acid sequence analysis by elevated temperature gas chromatographic technique, conventional gas chromatography, iodine value determination, ultraviolet spectrometry, $AgNO_3$ TLC, and others such as mass spectrography, NMR, X-ray diffraction, IR and laser spectroscopy for qualitative and quantitative determination of molecular lipids specifically. Using Nitrogen Molecule Laser in laser chromatography is preferred, the determination of qualitative and quantitative micro-analyses results in quick and accurate effects.

After being determined of their molecular structures and contents, the composition is then purified by means of molecular ultrafiltration and/or molecule-recrystallization, and then formulated in accurate weight in accordance with the composition of CHML of the invention, and mixed thoroughly at a temperature about 150,5-178 °C (303-353 K) for about 10-30 minutes. After reducing the temperature to about 142,3-147,8 °C (288-298 K) the mixture is passed through sintered glass filter to obtain the filtrate as a clear and transparent liquid of the CHML of the invention.

As a result of large amount of experiments, the prescribed composition of CHML has been developed by the inventor which possesses synergistic anti-cancer effects besides those of liposome carriers in the prior art.

The composition of the CHML provided in the invention is as follows:

Example 1 CHML

| | |
|---|---|
| Squalene | 0.5 - 2.0 wt.% |
| γ-linolenic acid | 0.7 - 2.5 wt.% |
| Linolenic acid | 1.0 - 4.0 wt.% |
| Oleic acid | 14 - 28 wt.% |
| Stearic acid | 5 - 10 wt.% |
| Palmitic acid | 6.5 - 12 wt.% |
| Eicosatetraenoic acid* | 3 - 7 wt.% |
| Eicosapentaenoic acid* | 8 - 11 wt.% |
| Eicosatrienoic acid* | 2 - 4 wt.% |
| Docosahexenoic acid | 10 - 15 wt.% |
| Tetracosenoic acid* | 2 - 4 wt.% |
| Vit E | 3 - 7 wt.% |
| Vit A | 0 - 0.5 wt.% |
| Vit D | 0 - 0.5 wt.% |
| Docosenoic acid | 4 - 6 wt.% |
| Docosadienoic acid | 0.5 - 2 wt.% |
| Docosatrienoic acid | 1 - 3 wt.% |
| Docosatetraenoic acid | 3 - 7 wt.% |
| Docosapentaenoic acid | 5 - 10 wt.% |
| Palmitoleic acid | 10 - 20 wt.% |

In example 1, * stands for that the components may be treated with bio-molecular activation, which will be clearly described in the following examples.

In processing the prescribed composition, additives such as surfactants may be selected to add with effect of "heterogenous rearrangement" of the prescriptive composition provided in the invention. The term "heterogenous rearrangement" in this invention means that the prescriptive composition is rearranged to the extent that the molecules of which should be arranged substantially into ordered orientation according to their polarities, and the polar radicals of the additives, such as the surfactants, metal ions or non-metal ions or other ions should come into combination with the molecular lipids such that the molecular lipids should be capable of affinity for the membrane structure of the abnormal cells and thus exhibit biological effect more significantly.

Additives, such as surfactants may be selectively added including Tween®, Span®, and other substances as glycerin, ether, ethanol higher alcohol sulfate (R--O-$SO_3$ Na), alkyl benzene sulfonate (R-$C_6$-$H_4$-$SO_3$Na), cholate, alkylamino acids (R-NH-$CH_2$-COOH), long chain alkyl quaternary ammonium salts, sodium lauryl sulphate, sulfated oils, polyoxyethylene fatty alcohol ether, polyoxyethylene alkylphenoxy ether, KOH, NaOH, $Ca(OH)_2$, KCl, NaCl, $CaCl_2$, $FeCl_3$, $FeCl_2$, $ZnCl_2$ $MgCl_2$, $MgSO_4$, KI, $NH_4Cl$, $NaHCO_3$, $ZnSO_4$, $Zn_3(PO_4)_2$, fluoroalkene, HCl, KBr, NaBr, KI and the radicals of molecules of alcohol-form, phenol-form, ether-form, aldehyde-form, and quinoid-form, and the radicals of amino acid glucose, pyrimidine, purine and any combination of them, etc. The best preferable composition of the additives comprises 10-90% glycerin, ether and ethanol, 90-10% higher alcohol sulfate, KOH, NaOH, fluorocarbon compounds such as FC-80 (perfluoro-butyltetahydrofuran, FDC polyfluoro-naphthalene, FTpA (trifluoro tripropyl amine), etc. More preferable composition of the surfactants comprises 70-30% (wt) Span®, Tween®, glycerine, and alcohol, with addition of 30-70% KOH, NaOH, hydrogenated castor oil, polyoxyethylene ether, ether and steapsin.

The steps for rearrangement of heterogenous molecular lipids can be illustrated by the following examples.

Example 2

At least one kind of additives as listed above e.g. KOH 30% aq. solution can be selected solely. It is warmed with addition of the molecular lipid components listed in Table 1, or 60% alcohol and 5% $MgSO_4$ can be selected as additives

also. Upon addition of molecular lipid solution, it is warmed and maintained at 283-340 K. The hydrophilic and lipophilic balance (HLB) is controlled at 3-16, pH 2-12, for 10-120 min under stirring, until its density is very uniform. The thoroughly mixed solution attains transparent features. It is a very finely dispersed suspension in which its molecular arrangement is very neat, that is the heterogenous molecular lipid product.

The formulated preparations of the above heterogenous molecular lipids can be prepared. They are illustrated in the following examples.

Example 3.

The selected additives such as surfactants are added to the freshly sterile water (for injection) and warmed together to 139-178 °C (283-363 K) to dissolve the additives, above accurately weight formulated components are added dropwisely to the mixture at same temperature, stirred thoroughly, a suitable amount of injectional water is further added according to its desired concentration, mixed and stirred to become a transparent clear solution, the heterogenous rearrangement is attained.

It is filtered through 0.025µ filtering membrane, perfused and sealed into ampule under vacuum or nitrogen atmosphere, sterilized at 189 °C (373 ± 1 K) with live steam for 30-60 min stored in the cold place and protect from light and heat, ready for use.

Example 4.

For adaptation of preparation of higher dosage, the mixture in example 1 is further homogenized at a high speed emulsifier, the pumps of emulsifier runs at 54 L/hr, working pressure 2000-8000 psi. The hydrophilic and lipophilic ratio (HCB) is maintained at 4-18. When the solution is mixed uniformly, its temperature is decreased to 145-150,5 °C (293-303 K), filtered through 0.6µ membrane 0.2-2% active carbon is added to the filtrate heated to 189 °C (373 ± 1) K boiling for 30-60 min, the temperature is decreased to 142,3-105,5 °C (288-303 K), filtered through 0.45 membrane, the filtrate is centrifugated by ultracentrifuge at 8000-50,000 rpm. a=15000-6000, for 5-10 min., finally the centrifugate is further filtered through 0.025 membrane, to obtain a clear transparent CHML filtrate, i.e. the heterogenous rearrangement is attained, perfused and sealed into ampule under vacuum or nitrogen atmosphere, sterilized with live steam at 189 °C (373 ± 1 K) for 30-60 min stored in protection of light and in cold place, ready for use.

The components and formulations of the cytotropic heterogenous molecular lipid CHML are described as above. The components can be derived from cell of plants and animals.

The source of cells of plants are taken from corn, peanut, seed of sunflower, cotton seed, soy bean, horse bean, vigna saicensis, garden pea, pheseolus radiatus, tea seed, mung bean castor seed, cocoa bean, sesame, olive, seeds of Chinese vegetable tallow, pumpkin seed, seed of sponge gourd, white gourd, pine seed, coconut, rape seed etc. as well as their stemes and leaves. Source of animal cells are taken from chicken, duck, goose, sparrow, cow, sheep, horse, pig, dog, deer, rabbit, tortoise and turtles and from marine organisms such as clam, scallop, oyster, shark, whale, cod, etc. The membrane of eggs of hen, duck and goose also can be used.

The components used in CHML are highly contained in the liver, heart, brain marrow, nerve and adrenal gland of animals.

The main source of various components contained in CHML of this invention may be obtained from plants, such as the fruit of corn, sunflower, sesame, peanut, rape seed, soy bean or may be obtained from marine organisms such as scabbard fishes, carcherhenidea spp such as blue shark and white shark. Gadus spp. such as cod, and the mammals such as whale and the like.

The animals and plants to be used must be selected in health, no genetic disease. The animals must have a certain extent of immunity. The animal organs to be used such as liver, kidney, heart, lungs spleen and brain must be picked in accordance with specific requirement, various components of saturated and unsaturated molecular lipids can be extracted from these organs according to the conventional art. For example the plants are selected finally, washed and dried, crushed into small sizes, warm pressed to get the crude vegetable oil or extracted with extracting agent as ether acetone etc. the crude oil vegetable product can also be obtained.

The vegetable oil as above for edible and medical use can be obtained from the market also. They can be used after their contents are determined, another example is: the corresponding animal tissue can be picked from animals under aseptic conditions and stored in 123 °C (253 K) refrigerator or the fresh tissue may be directly used.

The tissue of the animals is subjected to remove of impurities, crushed to small sizes, warmed at 150,5-161,5 °C (303-323 K) for 45 min then centrifugated under 2000 rpm for 10-20 min. The residue is warmed in distilled water (30% of original tissue wt.) kept the temp. for 20 min, further centrifugated under 2000 rpm for 10-30 min, the supernatant is discarded, distilled water (80% of original tissue weight) is added with stirring, the animal oil is separated by means of a separating funnel, that is the crude animal lipid.

When the crude lipid is extracted from vegetables and animals, various techniques such as saponification, refrigerated centrifuge, or adsorption may be applied to separate the saturated and unsaturated fatty acids.

Example 5.

The vegetable oil is subjected to saponify, the saponified liquor is maintained at 161,5 °C (323 K), then is acidified with 1:1 (V/V) HCl solution to pH 3.0, settled, the aqueous layer is discarded, the oily layer is collected and the emulsified layer is extracted with ethyl ether, combined the oil layer and ether extract, evaporating off ether, 10 fold amount acetone solution is added to dissolve remainder mixture. It is further subjected to fractional refrigeration according to their different freezing point of saturated fatty acid. It is freezed about 2 h, refrigerated centrifuge at 600-2000 rpm, for 5-10 min after the collection of various saturated fatty acids, evaporating off acetone, the mixed unsaturated fatty acids can be obtained.

Example 6.

3 fold amount of 95% ethyl alcohol is added to the homogenated suspension of shark liver, then filtered the suspension, the residue is further extracted with 2 fold amount of 95% ethanol, and filtered, the filtrate is combined and concentrated to remove alcohol.

Suitable amount of ethyl ether is added to the concentrated filtrate to remove insoluble matters and evaporated ether, thus the lipid is obtained.

It is dissolved in 1.5 fold of ethanol, pH of the lipid solution is adjusted with 50% KOH to above 10, saponified under nitrogen atmosphere, after its saponification is completed, filtered to separate the kali salts of mixed fatty acids and unsaponified lipids. Its temperature is further decreased to 112,5-117 °C (233-243 K), to facilitate the separation further.

The saponified solution is acidified with 6N HCl or 50% $H_2SO_4$ to pH = 3.0, extracted with ether, water layer is discarded, the ether layer of mixed fatty acids obtained is subjected to conc, of half of its original volume, stored in 123 °C (253 K) refrigerator for 24 h filtered with filter paper at 123 °C (273 K), the residue is further washed with ether, combined the filtrate and washing solution concentrated to half volume and stored in 123 °C (253 K) refrigerator for about 24 hours, filtered again. This operation can be repeated several times to separate and collect the solid saturated fatty acids and liquid unsaturated fatty acid. The components of solid fatty acid can be separated according to their different freezing points.

Example 7.

Dry silica gel 85 parts and calcined gypsum 15 parts (premixed with CMC 0.5-100%, and starch 10-40%) are mixed thoroughly to form dry silica gel-calcined gypsum mixed powder. 3-4 parts water is added to the part of powder and triturated to form a paste, and coated onto a glass plate to form a thin layer, thickness of this layer may be 250 milli-micron (nm). The adsorbing capacity is about 10-15 $mg/cm^2$. The coated glass plate is dried at ambient temperature, activated at temperature 178-195 °C (353-383 K) for 1 hour.

100% pure $AgNO_3$ is dissolved in small amount of water, to form a 10%-90% $AgNO_3$ solution. The plate with silica calcined gypsum thin layer are soaked into this solution for 30-120 sec, and dried in dark. The saponified lipid solution (after acidified) is adsorbed on the plate, after adsorption some non-polar solvents such as petroleum ether or hexane can be used as eluent to elute the fatty acids, the elute is treated with physiological salt solution to remove Ag ion, and subjected to separation to give various unsaturated fatty acids (with different ethylenic linkages). The saturated fatty acids may be collected from the eluate simultaneously.

As to the separation of various unsaturated fatty acids various techniques can be applied such as adsorption ultracentrifugation, ultrafiltration, freeze crystallization, chromatography, distillation, electrophoresis, ion-exchange, and the like. Other methods also can be used such as bromination-debromination, enveloped with urea, partitional solubilization, formation of $AgNO_3$ complex. EDTA-Na composite method.

Some composite methods are illustrated in following examples.

Example 8.

The mixed unsaturated fatty acids are dissolved in a suitable amount ot methanol solution, urea is added in the proportion: unsaturated fatty acid: urea: methanol = 1:3:7 (in weight), and stirred to dissolve urea, cooled under dry ice or liquid nitrogen and freeze crystallized according to the different freezing point of unsaturated fatty acids e.g. at 39-134 °C (103-273 K), filtered, the filtrate is dried over anhydrous $Na_2SO_4$, evaporating off methanol, various unsaturated acids can be separated.

Example 9.

The methyl ester of various unsaturated fatty acids are first prepared. 10 fold amount of 1mol/L of alcoholic KOH is added to the methyl ester, heated to reflux under N at 156-178 °C (313-353 K) water bath for half an hour.

After hydrolysis is complete, it is concentrated at reduced pressure to remove a portion of $CH_3OH$, 3 fold amount of water is added to it, acidified with 4 mol/l HCl to pH 3.0, extracted with ethyl ether. The ether layer is washed with water to pH 5-6, dried over $Na_2SO_4$, and evaporated under $N_2$ and reduced pressure. After the ether is drived, the residue is subjected to freezed crystallisation, to get various pure unsaturated fatty acids, kept under nitrogen, cold storage with protection of light.

As for liposoluble Vit A, D and E can be obtained from market, they also can be extracted from unsaponified lipid solution, but their cholesterol must be separated prior to extraction and the content must be determined before application.

In the preparation of CHML of this invention, the ultraviolet light and laser is used to convert the trace amount of cholesterol in the components into molecular lipid squalenes. The molecular lipid squalenes can't be conveniently obtained directly, but it can be as obtained by activation of cholesterol solution with uv light or laser, thus these kinds of molecular cholesterol lipid can be readily obtained with an economic procedure and cholesterol (with side effect to human body) can be converted into molecular lipid which is benefit to human body. That is the important and peculiar features in this invention, for example.

Example 10.

The double bond on gamma-site of cholesterol can be dehydrogenated by means of light energy, cholesterol can be converted into squalene. The synergistic effect of injury to cancer cell is enhanced about 50% by the CHML in this invention with application of these methods.

Chemical reaction is as follows:

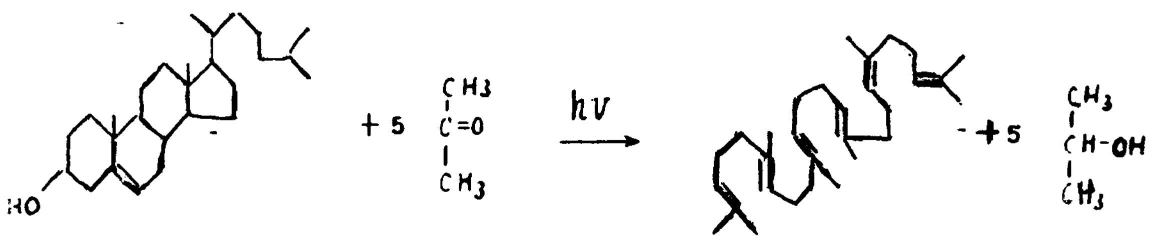


Cholesterol + acetone $\xrightarrow{h\nu}$ squalenes + secondary alcohol

Procedure. The unsaponified matters of animal oil and vegetable oil are extracted with acetone to get the cholesterol solution. 100 ml of this solution (or other acetone solution of cholesterol) are irradiated with 30-100 w ultra violet light lamp under atmospheric pressure directly, wave length of ultraviolet light = 2357 Å

$$\text{energy density} = 20\text{-}100\ \text{mw.sec/cm}^2,\ \text{irradiated time} = 50\text{-}10000\ \text{sec.}$$

Temperature = 159-175,6 °C (318-348 K), pH of solution is 4-10, relative humidity = 55%. Evaporating off secondary alcohols after irradiation, the molecular lipid with squalene components is obtained.

Applying laser irradiation, the molecular lipid of this invention can be activated to get the optimum biological effect. For example, eicosatrienoic acid, eicosatetraenoic acid and eicosapentaenoic acid are activated with laser, they can be converted to prostaglandin, vit. A can be converted to retinal, $\alpha$-tocopherol in vit. E can be converted to $\alpha$-tocopherol.

The injury of human normal cell by molecular lipid is reduced as small as possible when laser irradiation, which is applied in this invention, and its bio-availability in human body is enhanced.

For example, docosahexenoic acid provides the anti-hypertension effect to human, but it can injure the endothelial cell of blood vessel, the dissolution of cell may be occured. In the preparations of this invention with the application of laser irradiation, the long chain unsaturated molecular lipid can be cyclized at gamma-site double bond in small portions, not only the requirement of human is attained, but the injury of normal cell can be reduced also. As the polyenic unsaturated fatty acid with 10-30 carbon atom can be hydrogenated and cyclized at the gamma-site double bond by means of laser irradiation.

The laser used in this invention preferably are: ruby laser, Nd glass-laser transmitter $Nd^{+3}$ laser, YAG laser, He-Ne laser, $CO_2$ laser, argon ion laser, nitrogen molecular laser and helium-cadmium laser and the like.

The application of laser irradiation may be performed along with animal microsome enzyme, prostaglandinase, vit. C etc. for example.

Example 11.

The molecules of docosahexenoic acid (DHA) can be hydrogenated and cyclized by means of microsomelenzyme of sheep's seminal vesicle and laser irradiation.

The reaction scheme is as follows:

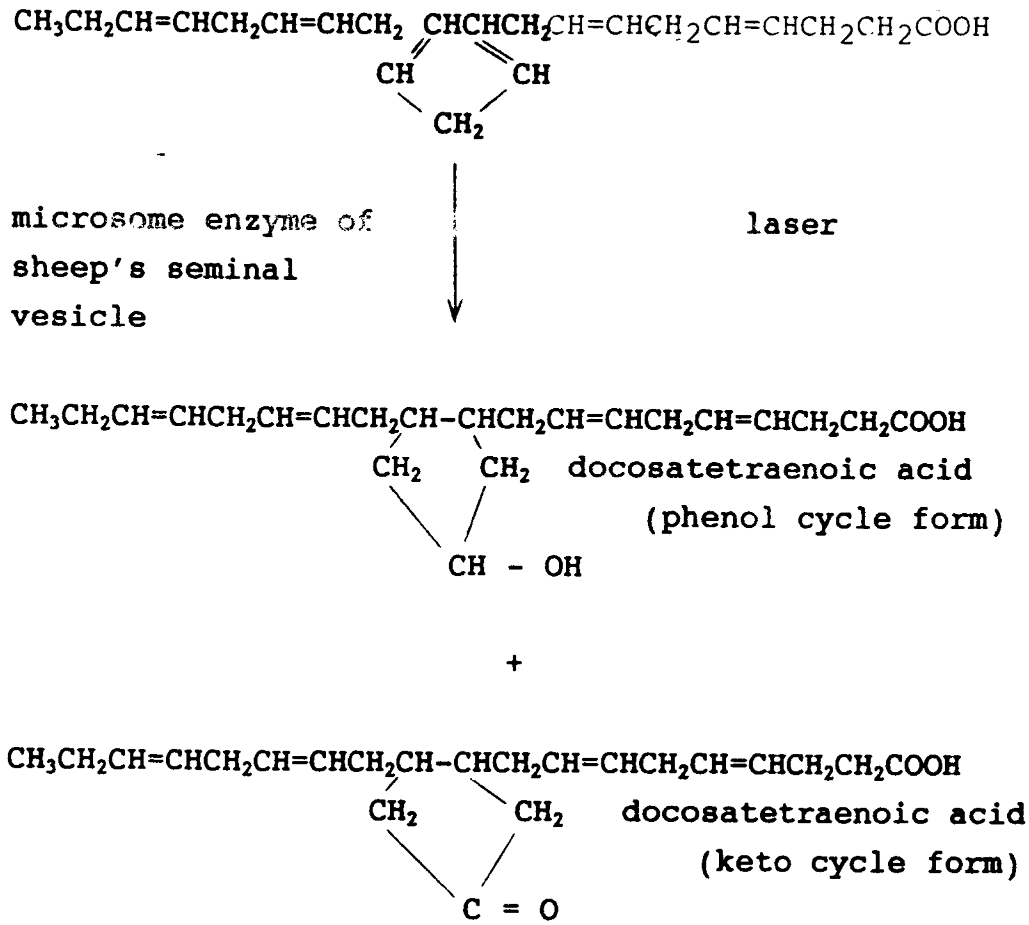


Procedure:

First step. Preparation of enzyme solution:

Sheep's seminal vesicle is taken from cold storage at 111,5-123 °C (233-253 K), its fat and connective tissues are removed, 1 l. 0.154M of KCl solution is added per kg of seminal vesicle, crushed and homogenized to give a homogenized suspension, centrifugated at 600 rpm for 10 minutes the supernatant is discarded, further centrifugated at 10,000 rpm for 20-40 minutes, acidified with 2 M citric acid to pH 4.6-5.6, centrifugated at 10,000 rpm, for 30-60 minutes, the supernatant is discarded.

The sediment is washed out with 100 ml 0.2 M phosphate buffer (pH 8), EDTA-2Na solution is added to the mixture, 2 M KOH is added dropwise to pH 7.5-8.2, cold storage in refrigerator, at 111,5-117 °C (233-243 K), that is the enzyme preparation.

Second step: The molecules are cyclized with hydrogenization:

Suitable amount of hydroquinone and glutathione are dissolved in a small amount of water and added into the 1 l enzyme preparation. 1-15g docosahexenoic acid (DHA) is added to the preparation of 1 kg sheep's seminal vesicle, under stirring and bubbling with oxygen. It is subject to laser irradiation with He-Ne laser (wave length 6328Å) power density: 0.1-50 mw/$cm^2$, depth of solution = 0.63-6.22 mm, irradiation time: 5-60 min, temp. 154,4-155 °C (310-311 K).

After the reaction is ceased, the heated mixture is cold slowly, 2 mol/L hydrochloric acid is added along the wall of the apparatus to pH 4.0-4.2, centrifugalized at 10,000 rpm for 30 min. To the sediment is added 3 fold amount of acetone, adjusted to pH 7 with aqueous ammonia, crushed and homogenized for 10-30 min. filtered under suction, pressed to dry, this operation is repeated in several times to get the filtrate. 2 mol/l phosphate buffer is added to the filtrate to adjust pH to 8, the aqueous layer is defatted with petroleum ether, acidified with 2 mol/lHCl to pH3.0, extracted with $CHCl_3$ extracts are washed with water to remove the free acid, evaporating off $CHCl_3$, cyclic docosahexenoic acid is produced, then subjected to chromatographic purification by means of $AgNO_3$-silica gel as adsorbent, ethyl acetate as developer, the eluent obtained is treated with physiological salt solution to remove Ag+, dried over anhydrous $Na_2SO_4$, filtered and the filtrate is concentrated under $N_2$ atmosphere and reduced pressure, ethyl acetate is removed, lyophilized to get pure cyclized docosahexenoic acid products.

The molecular lipids obtained as above are precisely analyzed with the technics of u.v. spectrometry, gas chromatography, mass spectrography, nuclear magnetic resonance, infrared spectrometry and laser spectrum analysis for determination of their molecular structure, and purity. Then various molecular lipids are purified by means of their fine filtering and recrystallization.

Finally according to their necessities of various bio-effect. They can be formulated into different preparations. The formulation included accurately weighed components of molecular lipid mixed with other molecular lipids, to which suitable amounts of additives as above are added, thoroughly mixed at 150,5-178 °C (303-353 K) for 10-30 minutes, decrease temp. to 142,3-147,8 °C (288-298 K), filtered through sinter glass filter. The filtrates can be directly used as the CHML of this invention. They have good quality and excellent effects.

CHML of this invention can be made in various preparations used for synergetic treatment or prophylaxis of cancer. CHML can be used as carrier of some antitumor drugs, such as 5-fluorouracil, Ping Yang mycin in administration and exert the synergetic effect of "1st attack" and "2nd attack" to the cancer cells.

The medicated forms, starting materials, dosages of medicaments administration for and method of their preparations are illustrated in the following examples.

Example 12.

Tablet: It can be used mainly in treatment and prophylaxis of the pathologic change to cancer at earlier stages such as carcinoma of stomach, colon and ractal cancer etc.

Formulations:

| Materials | Amount used per 160 tablets |
|---|---|
| CHML | 50 ml. |
| dextrin | 17.8 g |
| sugar powder | 10.2 g |
| talc powder | 1.5 g |
| magnesium stearate | 1.0 g |
| antitumor drugs | suitable amount is necessary |

Method of the preparation: CHML is concentrated to 20-40% level, to which the antitumor pharmaceutical, sugar powder, and starch are added and groud together to form a paste, it is further mixed with other components, sieved to pass 160 mesh and pressed into tablets.

Example 13.

Micro capsules: It is adapted for the synergetic treatment to the various systemic cancer and for prophylaxis of cancer at earlier stage.

The dry small size micro capsules are soaked in the 10% CHML solution, after CHML is absorbed into it, and dried in a drying box. CHML-micro capsule preparation is obtained, ready for use.

Example 14

Eye drops

It may be adapted for synergetic treatment and prophylaxis of the eye disease infected with virus and precancerous stage cancer and carcinoma in eyes.

Formula 1.

| Material | Amount |
|---|---|
| CHML | 50 mg |
| anhy. $Na_2HPO_4$ | suitable amount |
| anhy. $NaH_2PO_4$ | " |
| moroxydine hydrochloride (ABOB) | 4 mg |
| distilled water | added to 100 ml |

Formula 2.

| Material | Amount |
|---|---|
| CHML | 100 mg |
| boric acid | suitable amount |
| borax | " |
| antitumor drugs | as necessary |
| distilled water | added to 100 ml. |

CHML and phosphate are mixed and warmed in small amount of distilled water, borax, boric acid, moroxydine hydrochloride or antitumor pharmaceutical are added and mixed with suitable amount of distilled water to the predetermined volume, perfused separately into dried, sterilized dropping bottles for eye drops.

Example 15.

Injection: the injection is adapted to synergetic treatment and prophylaxis of various benign tumor, malignant tumors (or carcinoms) at the precancerous stage. It is administrated in local injection, i.v. injection and intravenous drop in the form of CHML preparations. They may contain or not contain antitumor pharmaceutical, determined by the clinical physician.

Preparations for local injection can be injected into the zone of pathologic change directly.

| Materials | Amount |
|---|---|
| CHML | 10 g |
| ethanol (95%) | 2 ml |
| antitumor drugs | suitable amount as necessary |
| distilled water | added to 100 ml. |

CHML is mixed thoroughly with distilled water and ethanol, then perfused and sealed in ampoule (1 ml or 2 ml).

Specification: Each ampoule is perfused with 1 ml CHML preparation or 2 ml CHML preparation, sterilized by live steam at 373 K, ready for use.

The injection for i.v. and intravenous drip: The injection for i.v. is characterized in the synergetic therapy of strongly pathogenic change and systemic transference of cancer, e.g. according to the suggestion of the results of examination of freezed pathological section during the operation, it be suited the exigent treatment. The intravenous drip may be applied to the treatment and prophylaxis of leukemia. The content of CHML can be 5%, 10%, 15% and 20% respectively, material and dosage are same as above this example. When it is used in i.v. injection, 1 ml of CHML preparation is diluted with 20 ml physiological salt solution as for intravenous injection, 1-2 ml of fresh CHML injection is diluted with 5% glucose containing physiological salt solution for intravenous drip.

Example 16. Aurinasal drops.

It is applied to synergetic therapy and prophylaxis for the precancerous stage and cancer in nasal cavity and ears, especially for treatment of nasopharyngeal carcinoma.

| Materials | Amount |
|---|---|
| CHML | 250 mg |
| peppermint crystal | 1 g |
| ping yang mycin | suitable amount as necessary |
| liquid paraffin | added to 100 ml. |

CHML, suitable amount of Ping Yang mycin, peppermint crystals are put together in a mortar, liquid paraffin is added, ground and mixed thoroughly, liquid paraffin is further added to the required volume, ready for use.

Example 17. Aerosol:

It is applied to synergetic therapy and prophylaxis for lung cancer, is applied to synergetic therapy and prophylaxis for bronchial cancer and esophageal cancer.

| Materials | Amount used for 20 bottles of aerosols |
|---|---|
| CHML | 60 ml |
| lemon essence | 1 ml |
| 7% ethanol | 56 ml |
| saccharin sodium salt | 4 g. |
| 5-fluorouracil (5-Fu) | suitable amount as necessary |
| dichloro difluoro ethane | 160 ml. |

CHML and 7% ethanol are warmed together to 156-167 °C (313-333 K), cooled to 139 °C (283 K) subsequently, lemon essence and 5-Fu are added, thoroughly mixed and perfused into aerosol bottle separately. Dichloro difluoro ethane is pressed with it, sealed, ready for use.

When CHML in this invention be used as carriers with carrying a small amount, or even very small amounts of drugs, it can exert the strong synergistic effect. It is so called "bio-molecular missile". It has been confirmed by a lot of pharmacologic research works. For example, inhibition to S-180 Sarcoma cells in vivo, effects on B-cell, preclinical pharmacologic research work, Ames test, determination of conventional dosage and limitary dosage, etc.

I. The experiment of inhibitory effects of CHML on S-180 Sarcoma cells

CHML may obliterate whole S-180 sarcoma cells in vitro. This may show strong bio-effect of CHML.

Materials:

CHML provided by inventor, 0.5% content
Incubating liquid RPM1-1640 (liq.) S-180 Sarcoma cell provided by Shanghai Cancer Institute.

Method:

The mice was inoculated with S-180 cells and raised for 7 days. They were killed by drawing their necks. S-180 cells containing ascite were drawn out, a small amount of RPMI-1640 was added, the numbers of S-180 cells in ascite are counted. RPMI-1640 was further added to adjust the cell content in 5 x $10^6$ /ml. 1.5 ml of above incubating liquid was transferred to each test tubes (total = 10 tubes).

5 tubes served as control and 5 tubes for examination of experiment. RPML-1640 incubating liquid was dropped in 0.2 ml/tube for control and 1 mg of CHML was dropped into each tube containing the incubating fluids, covered, reposited in 37°C thermostatic water bath, centrifugated over every 10 min interval,the sediment was taken and examined smears with wright's staining method. This experiment was performed in triplicate. The results were shown in Table 2.

Table 2

| groups | dosage | cell counting/ml | mortality of S-180 cell (%) | | | | |
|---|---|---|---|---|---|---|---|
| | | | 10 min. | 20 min. | 30 min. | 40 min. | 50 min. |
| control | - | 5 x $10^6$ | - | - | - | - | - |
| experiment | 1mg/ml | 5 x $10^6$ | 50 | 60 | 70 | 85 | 100 |

The morphological change of S-180 cells treated by CHML is shown in Fig. 3 (a) to Fig. 3 (d).

The dosage of appropriate components or concentrations of CHML can be selected for killing the cancer cell within 2-3 seconds, but it also exhibits the injury of human healthy cell, in certain extent. It is preferably to control the components and concentration of CHML to a certain level to injury of cancer cell and maximally decrease the effect for injuring to the human normal cells.

II. Preclinical pharmacological experiments of CHML on animals.

The sources of CHML of this invention are derived from the cells of normal plants and animals, their components are liquids acceptable by human body. According to the literature cited, their toxicities are very low under suitable amounts administered. The results obtained from toxicity test in animals are shown below.

1. Acute toxicity test (examinational period is two weeks after injection).

30 mice, average body weight is about 15 g. 2 months old mice are divided into 3 groups optionally, 10 mice for each. The control group are subcutaneously injected into the caudal of the mice with 5% glucose saline 1.5 ml. Whereas 2 experimental groups are injected into the caudal vein of mice with 25mg/kg and 50mg/kg CHML plus 5% glucose saline 1 ml respectively. Their mobility are examined. It is examined whether mice closure of hairs, refusal of food, marasmus gradually to death will appear or not, whether side effects of cardiovascule, respiratory tract, digestive tract will appear or not. The results of higher dosage group (experimental) suggested that 20 min to 48 h after treatment, only a slight effect has been observed in cardio vascule, respiratory tract, digestive tract in some mice, but mobility are kept in normal state, no relaxations or deluster of hairs observed after 48 h eat violently after 72 h. no marasmus and death occured. The side effect of lower dosage group are more slight, $LD_{50}$= 4876 + 196.4 mg/kg.

24 rabbits (average body weight 2.3 kg) are divided into 3 groups, 8 rabbits for each group. In the experimental group, 10-30ml of 1% CHML and 5% glucose saline 2 ml are injected (i.v.) into the subcutaneous vein (at the back of the ears) respectively and examined tha basal body temperation and local and systemic side effects. The basal body temperature is detected by a portal thermometer, average body temperature is 38.2 ± 0.2°C before injections and 38.2 ± 0.4°C in 48 h after injection. In the higher dosage group, a slight local blood clot is formed in the vein of 8 rabbits and slight tumefaction in the local site and local temperature is raised, when subjected to 24-72 h. tumefaction is disappeared, body temperature is in normal, no infections in tissue, no necrosis is observed. Systems examinations: respiration and heart rate is increased during injections for 30 minutes, slight decreased appetite is observed as the side effect of digestive tract, but appetite is enhanced after 72 hours, no abnormalities are observed at nerve system, the lustrousness of hairs are in normal. No marasmus and death are observed.

2. Subacute toxicity test. (examinatorial period 2 week to 6 months after injection): 30 rabbits, average body weight about 2 kg, female:male =15:15, are divided optionally in to 3 groups, the one as for control and another 2 groups as experimental, 10% glucose solution 3ml are injected (i.v.) into the subcutaneous vein (at the back of the ears) as control. 25 mg/kg and 50 mg/kg CHML plus 10% glucose solution 4 ml are injected (i.v.) into the subcutaneous vein (at the back of the ears) as experimental respectively, their blood pressure, heart rate, side effect in the nerve system and digestive systems are examined. Their body weight, hemoprotein content, erythrocyte counting, leukocyte counting, conventional analysis of urine, functions of liver and kidney and other organ are also examined by pathological methods and by nacked eyes.

Result of experimental group. The body weight and leukocyte counting are increased slightly except the blood pressure tends to decrease, heart rate increased after 48 h, no abnormalities are observed at the effect of digestive tract but appetite is enhanced after 72 hours, no death is occured.

The results of hemoprotein content, erythrocyte counting, leukocyte counting, conventional urine analysis, functions of kidney, liver and other organs are normal by examination of pathological slices and by eye inspections.

3. Special toxicity test (period for inspections 1 months to 6 months) 18 female mice (about 2 weeks prior to pregnancy) average body weight about 18 g, are optionally divided into 3 groups, 6 mice group, one group serves as control injected (i.p.) with 5% glucose saline 2ml/mouse, another two groups serves as experimental are injected (i.p.) with 50mg/kg and 100mg/kg CHML + 5% glucose saline 1 ml respectively. Examination: The newborn mice are examinated.

The results show no abnormality is observed in new born mice, the digestive tract and nerve system are normal, their developments are same as control group.

6 female dogs, average body weight 18.5 kg (about months postpregnancy) are divided into 3 groups optionally, one group serves as control, injected (i.p.) with 5% glucose saline 8 ml/day. Another 2 groups serve as experimental, injected with 100 mg/kg and 200 mg/kg of CHML and 5% glucose saline 4 ml respectively.

Examinations: The newborn dogs are examinated.

The results shown no abnormality is observed in newborn dogs, in the experimental group, their digestive tract and nerve system are in normal, developments are the same as control group.

4. Test for side effect of local site in animals:

(1) Test on skin

The results of the test for cutaneous side effect on mice (Kunming species) and rabbits show no side effect under the dosage of CHML 5% 20μl/mm$^2$.

(2) Test on nose-drops.

No side effect es appeared in the naso-mucosa of the mice under the dosage of 2.5% CHML 2 ml per mouse every time.

(3) Test of eye-drops.

No side effect is appeared in the eyelid conjunctiva and eyeballs of rabbits, under the dosage 0.5% CHML 0.1 ml/eye every time.

III. Ames test for CHML.

Identification of strains:

Histidine deficienly mutant strain of Salmonella typhimurium: TA98 and TA100 with R-factors (separately) are used as test strains, the strain is examined for the requirement of histidine for growth spontaneous reversion test, test for delation of repairing system (UV, B), test for deletion of barrier of lipo-polysaccharide (rf a) and the test for the resistance to ampicillin be used as standard test to examine proof strains.

The strains are inoculated in the broth culture at 37°C for 16 h. prior test. The viable count of the cell suspension must be maintained at $10^8$-$10^9$, it can be used for determination of mutagenesis.

Preparation of S-9 mixture:

Male rats (average body weight about 150 g) are administered with polychlorobiphenyl (Arochlor 1254) which is diluted with corn oil, dosage 500 mg/kg for 5 days. The rats are dissected and the livers are taken out and homogenized to get 9000g homogenized suspension. It is then subjected to centrifugalization, the supernatant is brought to process to S9. It is freeze.stored in liquid $N_2$.

During utilization S-9 mixture is made by addition of S-9 0.3 ml. $MgCl_2$ 8 µmol. KCl 33 µmol. G-6-P; 5 µmol NADP 4 µmol and pH=7.4 phosphate buffer solution 100 µmol per 1 ml of S-9 mixture. The amount of S-9 mixture used for examination is 0.2ml/dish (about S-9 40 µmol.) These operations must be carried out under aseptic condition and the microsome activated systems of rat's liver must be added during examination.

Control of mutagenesis

The CHML sample is diluted to a series of concentrations (5 µg - 500 µg/me) with dimethyl sulfoxide (DMSO), 3 Petri dishes are taken for each concentration for test. The basic incubating medium (U-B low concentration liquid) 15 ml are added to each dish. After coagulation (2 ml of upper layer incubating medium), 0.1 ml of bacterial suspension, sample of each concentration to be examined 0.1 ml (the concentrations of the sample per dish are 0.5, 5, 50, 500 & 5000 µg respectively) and S-9 mixture 0.2 ml are also added together into the upper layer of incubating medium 2 ml. After coagutation, they are incubated at 37°C for 48 hrs. The reversed mutagen colonies are countered.

Besides the spontaneous reversion the bacterial suspension added with S-9 only are served as negative control, the cyclo phosphamide and diacetylamino fluorene are used as negative control also.

Table 5-a

| Test sample | test dosage µg/dish | mutagenesis | |
|---|---|---|---|
| | | TA98 | TA100 |
| CHML | 0.5 -5,000 | - | - |
| Cyclophosphamide (CTX) | 1,500 | | + |
| diacetylamino fluorene (2AF) | 200 | + | + |
| spontaneous reversion | | - | - |

Table 5-b

| Test sample | test dosage µg/dish | numbers of reversion colonies (average) | |
|---|---|---|---|
| CHML | 0.5 | TA98 | TA100 |
| | | 26 | 203 |
| | 5.0 | 13 | 233 |
| | 50.0 | 18 | 146 |
| | 500.0 | 24 | 125 |
| | 5000.0 | 17 | 137 |
| Cyclophosphamide(CTX) | 1500 | | 519 |
| diacetylamino fluorene(2AF) | 250.0 | 254 | 286 |
| numbers of spontaneous reversion | | 28 | 155 |

Discussion:

These experiments as above have confirmed that there is no reversed mutation occured against test organisms by various components of CHML preparations in this invention, and further evidenced that the mutation of codons and the mutation of base pair replacement can't be induced also.

IV. Conventional dosage and limiting dosage of CHML. The results are shown as follows.

| Mouse (Kunming sp.) | | |
|---|---|---|
| i.v. administration | conventional dosage | 25 mg/kg |
| i.p. administration | limiting dosage | 150 mg/kg |

| Rabbits | | |
|---|---|---|
| i.v. administration | conventional dosage | 50 mg/kg |
| i.p. administration | limiting dosage | 400 mg/kg |

V. The phagocytosis test of alveolar macrophages in rabbits:

1. Extraction of alveolar macrophage

6 health rabbits (female:male=3:3) are chosen. Their average body weight is 3 kg. Their lungs along with distal trachea are extracted under anesthesia, suspended the lungs in a glass disc containing 0.01M PBS (phosphate buffer solution pH 7.4, 30 ml washing solution (pH 7.4 0.01M PBS) is perfused into trachea by means of a 50 ml syringe and drawn out again. This operation is performed 3 times. The amount of the drawn off liquids are recorded. Each liquid is combined, then perfused into a bottle, cold storage, ready for use.

Cell counting, above washing solution 0.5µl is placed on a glass slide, mixed well with equal amount of trypan blue added and dropped into a cytometer, the total number of alveolar macrophage is counted, according to the method of counting for the leukocyte.

The 1st recovery of washing solution is attained to 84.6% 2nd at 96.6% and 3rd at 98.3%. The total count of the rabbit's alveolar macrophages (6 rabbits) are 2.5x $10^5$ /ml, living cells = 93.5%.

There are alveolar, macrophage (average) 92.4% eosinocyte 6.5% lymphocyte 0.9%, neutrophil leukocyte 0.4% in the washing solution.

2. Bacteriol phagocytosis test.

Control group: 8 ml of washing solution are mixed with 4 ml B. Candida Albicens suspension (30,000,000 bac/ml), incubated at 37 for 30 min centrifugated 10 min at 100 rpm, the sediment is spread onto a glass slide to form smears, stained with Wright's staining method staining and H.E. staining, examined under microscope.

Experimental group:

8 ml of washing solution are mixed with 4 ml candida albicans suspension (3 x $10^7$ bac/ml) for 10 minutes, 5% CHML 10 ml, 10% CHML 10 ml are added separately incubated at 37°C for 20 minutes, centrifugalized at 1000 rpm for 10 min. The sediment is spread onto a glass slide to form smears, stained with Wright's staining method and HE staining, examined under microscope, to calculate and record the percentage of phagocytosis and phagocytic index as follows:

$$\text{percentage of phagocytosis} = \frac{\text{number of alveolar macrophage for phagocytosis of bacteria}}{\text{200 alveolar macrophage}} \times 100\%$$

$$\text{phagocytic index} = \frac{\text{number of bacteria phagocytosized by 200 alveolar macrophages}}{\text{200 alveolar macrophages}}$$

The results are shown in Table 8

Table 8

| groups | percentage of phagocytosis | phagocytic index |
|---|---|---|
| control | 6.3 ± 4 | 0.09 ± 0.07 |
| experimental | 10.0 ± 6 | 0.14 ± 0.08 |

VI. The effect of CHML on squamous carcinoma of human esophagus in vitro.

Material and methods:

Same as the experiment for killing of S-180 sarcoma cells with CHML preparations. The experiment are carried out in triplicates. The results are shown in Table 9.

Table 9

| groups | dosage | numbers of cancer cell | mortality of cancer cell(%) | | | |
|---|---|---|---|---|---|---|
| | | | 10 min | 20 min | 30 min | 40 min |
| control | --- | $5x10^5$ | --- | --- | --- | --- |
| experimental | 1mg/ml | $5x10^5$ | 60 | 80 | 95 | 100 |

It is evident that CHML of this invention is a series of "biomolecular missile". The results of the inhibition test of human cancer cells with CHML (in vitro) and a series of experiment provide a valid basis for the feasibility studies on the clinical trials.

According to the studies on mechanism; the degree of oxygen dissociation in tissue cells are decreased while the content of CHML is attained to 0.1 mole/g, but the ability of carrying oxygen by CHML is increased. When the CHML content is attained to 19 mole/g, the degree of oxygen dissociation in tissue cells is increased, the abilities of CHML for oxygen carrying is decreased significantly. During the mutations of tissue cells, suitable concentration of CHML entered into mutant cell, large amount of oxygen is absorbed by CHML. The oxygen deficiency within the mutant cell is made apparent, the oxidative phosphorylation of mitychondrion is blocked, ATP decreased, and the synthesis of protein nucleic acid is blocked, the binding of Ca to CHML is increased. The function of endoplasmic reticulum is blocked. Thus the mutation of cells can be inhibited.

VII. Studies on pharmacokinetics of CHML preparation in animals.

16 health mice (kun-ming sps.) are selected and optionally divided into 4 groups. 4 mice per group, average body weight about 20 g. The experimental groups is injected into to caudal vein (I.V.) with 25 mg/ml. 99mTc- CHML 0.5 ml. 0.5 ml of physiological salt solution is injected in the control group. After injections, during the interval of 2 h, 24 h and 72 h, the blood 0.5 ml is taken from their orbits. and then liver, spleen, lung, stomach and brain tissue are dissected, accurately weighed and put into test tubes to determine the intensity of radiation of every tissue by scintillation counter.

The results are expressed in the radioactive pulse percentage per gram of various organ tissue occupies the pulse percentage of all organ tissue. These results are shown in Table 14.

Table 14

| groups | total pulse percentage of every tissue | | | | | | | |
|---|---|---|---|---|---|---|---|---|
| | pulse % | blood | liver | spleen | kidney | lung | stomach | brain |
| control | --- | --- | --- | --- | --- | --- | --- | --- |
| 2 hrs. | 100 | 9 | 65 | 16 | 4 | 4.5 | 0.5 | 1 |
| 24 hrs. | 67.2 | 4 | 40 | 12 | 8 | 3 | 1.2 | --- |
| 72 hrs. | 6 | --- | 3 | 2 | 1 | --- | --- | --- |

The results of animal tests shown in Table 14 show after i.v. injection of CHML 2 h, the pharmaceutical distributes into liver, spleen, kidney, lung and brain in 2 h. The absorption in liver is highest. The next are in lung, spleen and kidney. The levels in the stomach and brain are in minimum. After 24 h. metabolism products of CHML excreted from the kidney are about 32%, but there is a high retention in liver and spleen. After 72 h metabolism products of CHML excreted from body are 94%, only trace amount retained in liver, spleen and kidney.

VIII. Antitumor effects of CHML in vivo test.

1. Effect of CHML on S-180 ascites sarcoma

Material: CHML provided by inventor
Mice BalB/C, Kunming sp. are provided by Shanghai Second Medical University.
S-180 sarcoma strain provided by Shanghai Cancer Institute

Method: Mice are inoculated with S-180 ascites sarcoma strain according to the conventional method, and administered 0.5% CHML by stomach perfusion. 8 times per day for 8 days continuously the ascites sarcoma are weighed and compared with control, the results are shown in following.

Results:

| groups | dosage (days) mg/kg | number of mice initial/final | body weight (g) | sarcoma weight (g) | inhibition rate (%) | P |
|---|---|---|---|---|---|---|
| control | -(x8)PO | 20/20 | 20.0/27.1 | 1.69 | --- | -- |
| experimental (1) | 500(x8)PO | 8/8 | 20.0/25.1 | 0.75 | 56 | <0.01 |
| experimental (2) | 350(x8)PO | 8/8 | 20.5/26.4 | 0.81 | 52 | <0.01 |
| experimental (3) | 250(x8)PO | 8/8 | 20.6/26.7 | 0.94 | 44 | <0.01 |

2. Effect of CHML on S-180 solid sarcoma

Material: CHML is provided by inventor
Mice BalB/C Kunming sp. is provided by Shanghai Second Medical University
S-180 sarcoma strains provided by Shanghai Cancer Insti tute

Methods: Mice BalB/C are inoculated with S-180 sarcoma strains according to the conventional method, and administered with 0.5% CHML by stomach perfusion, 8 times per day for 8 days continuously. The mice are dissected and the sarcoma are taken out, weighed and compared with the control. The results are shown as following.

Results:

| groups | dosage (days) mg/kg | number of mice initial/final | body wt.(g) initial/final | sarcoma wt. (g) X+SD | inhibition rate (%) | P* |
|---|---|---|---|---|---|---|
| control | -(x8)PO | 18/18 | 21.0/25.3 | 2.17±0.53 | --- | -- |
| experimental(1) | 500(x8)PO | 8/8 | 20.4/27.3 | 0.86±0.32 | 60.4 | <0.01 |
| experimental(2) | 350(x8)PO | 8/8 | 20.4/06.0 | 1.14±0.44 | 47.5 | <0.01 |
| experimental(3) | 250(x8)PO | 8/8 | 20.3/25.4 | 1.41±0.59 | 35.0 | <0.01 |

3. Effect of CHML on S-180 solid sarcoma by administration in local injection

Material same as above

Method: Mice BalB/C are inoculated with S-180 sarcoma cells according to the conventional method, and administered with 0.5% CHML by injected into the local site (duration: 4 times per day, administered period = 8 days), the mice are dissected, the sarcoma are taken out, weighed, compared with the control.

Results:

| group | dosage (days) mg/kg | number of mice initial/final | body wt. initial/final (g) | wt. of sarcoma (g) | inhibition rate % | P |
|---|---|---|---|---|---|---|
| control | -inj.in local site | 18/18 | 20.4/28.3 | 1.30±0.61 | --- | -- |
| experimental (1) | 500 " | 8/8 | 21.0/25.3 | 0.37±0.32 | 82.6 | <0.01 |
| experimental (2) | 350 " | 8/8 | 20.5/26.1 | 0.47±0.70 | 77.9 | <0.01 |
| experimental (3) | 250 " | 8/8 | 20.8/26.6 | 0.66±0.53 | 69.0 | <0.01 |

Discussion:

The therapeutic effects in animals are shown in above results by the CHML preparation in this invention in vivo. It provides significant antitumor effects. The inhibition rates are depend upon the dosage administered.

The results show that the CHML with very small sizes can attack to the cancer cell to become fragments. It is further proved that the CHML provides the function of molecular missile in its actual effects.

From the large amount of experimental results, the "molecular missile" CHML in this clinical trial is an effective pharmaceutical carrier, not only in theoretical consideration but the actual effects appeared also. For example, CHML molecular lipid bind to the membrane of cancer cell specifically, cancer is due to the function of sodium pump of the membrane of cell is in sthenic state, micro viscosity is increased, CHML can attack accurately to the target cell. According to the above experimental results and photographs, it can be seen that CHML with very small sizes can attack to the cancer to become fragments. It is further proved that the CHML provides the function of "molecular missile" in its actual effects.

Descriptions as above, include all the special terms, examples and figures, there is only for illustration of specifity of this invention in some extent. This total description is only for illustration and by no means of limitation.

However, it can be understood that there may be many modifications and variations in the changes of different forms, sizes, structures, components, purities and compositions of the present invention. However, applicant do intend to include all such obvious modifications and variations within the scope of the invention which is defined by the following claims to be protected.

Illustration for figures:

Fig. 1. photograph of transmission electron microscope, (JEM 200 CX, 100 kV) it shows the molecular size of CHML, CHML molecule is shown by the arrow head. Amplification: 525000 times.

Fig. 2. photograph of CHML product, CHML is a transparent liquid, this product has been stored for 2 years.

Fig. 3. photographs for the anti-S-180 sarcoma cell test.

(a) smears of control: S-180 sarcoma cell counting = $5x10^6$/ml 50 min x 350.
(b) smears of experimental tube:
S 180 cell counting = $5x10^6$ /ml, 1 mg of CHML solution is added. 20 min x 350.
(c) smears of experimental tube:
S 180 cell counting = $5x10^6$ /ml, 1 mg of CHML solution is added. 40 min x 350.
(d) smears of experimental tube:
S 180 cell counting = $5x10^6$ /ml, 1 mg of CHML solution is added. 50 min x 350.

All of the S-180 sarcoma cells are attacked, and turned to fragments.

**Claims**

1. A pharmaceutical composition which comprises:

0.5 - 2.0 wt. % squalene;
0.7 - 2.5 wt. % γ-linolenic acid;
1.0 - 4.0 wt. % linolenic acid;
14 - 28 wt. % oleic acid;
5 - 10 wt. % stearic acid;
6.5 - 12 wt. % palmitic acid;
3 - 7 wt. % eicosatetraenoic acid;
8 - 11 wt. % eicosapentaenoic acid;
2 - 4 wt. % eicosatrienoic acid;
10 - 15 wt. % docosahexenoic acid;
2 - 4 wt. % tetracosenoic acid;
4 - 6 wt. % docosenoic acid;
0.5 - 2 wt. % docosadienoic acid;
1 - 3 wt. % docosatrienoic acid;
3 - 7 wt. % docosatetraenoic acid;
5 - 10 wt. % docosapentaenoic acid;
10 - 20 wt. % palmitoleic acid;
3 - 7 wt. % Vitamin E;
0 - 0.5 wt. % Vitamin D; and
0 - 0.5 wt. % Vitamin A.

**Patentansprüche**

1. Pharmazeutische Zusammensetzung die folgende Komponenten umfaßt:

0,5-2,0 Gewichtsprozent Squalen,
0,7-2,5 Gewichtsprozent γ-Linolensäure,
1,0-4,0 Gewichtsprozent Linolensäure,
14-28 Gewichtsprozent Oleinsäure,
5-10 Gewichtsprozent Stearinsäure,
6,5-12 Gewichtsprozent Palmitinsäure,
3 - 7 Gewichtsprozent Eicosatetraensäure,
8 - 11 Gewichtsprozent Eicosapentaensäure,
2 - 4 Gewichtsprozent Eicosatriensäure,
10-15 Gewichtsprozent Docosahexensäure,
2 - 4 Gewichtsprozent Tetracosensäure,
4 - 6 Gewichtsprozent Docosensäure,
0,5-2 Gewichtsprozent Docosadiensäure,
1 - 3 Gewichtsprozent Docosatriensäure,
3 - 7 Gewichtsprozent Docosatetraensäure,
5 - 10 Gewichtsprozent Docosapentaensäure,
10-20 Gewichtsprozent Palmitoleinsäure,
3 - 7 Gewichtsprozent Vitamin E,
0 - 0,5 Gewichtsprozent Vitamin D und
0 - 0,5 Gewichtsprozent Vitamin A.

## Revendications

1. Composition pharmaceutique qui comprend :

   0,5 à 2,0 % en poids de squalène ;
   0,7 à 2,5 % en poids d'acide γ-linolénique ;
   1,0 à 4,0 % en poids d'acide linolénique
   14 à 28 % en poids d'acide oléique ;
   5 à 10 % en poids d'acide stéarique ;
   6,5 à 12 % en poids d'acide palmitique ;
   3 à 7 % en poids d'acide eicosatétraénoïque (ou acide arachidonique) ;
   8 à 11 % en poids d'acide eicosapentaénoïque ;
   2 à 4 % en poids d'acide eicosatriénoïque ;
   10 à 15 % en poids d'acide docosahexénoïque ;
   2 à 4 % en poids d'acide tétracosénoïque (ou acide nervonique) ;
   4 à 6 % en poids d'acide docosénoïque ;
   0,5 à 2 % en poids d'acide docosadiénoïque ;
   1 à 3 % en poids d'acide docosatriénoïque ;
   3 à 7 % en poids d'acide docosatétraénoïque ;
   5 à 10 % en poids d'acide docosapentaénoïque ;
   10 à 20 % en poids d'acide palmito-oléïque ;
   3 à 7 % en poids de vitamine E ;
   0 à 0,5 % en poids de vitamine D ; et
   0 à 0,5 % en poids de vitamine A.

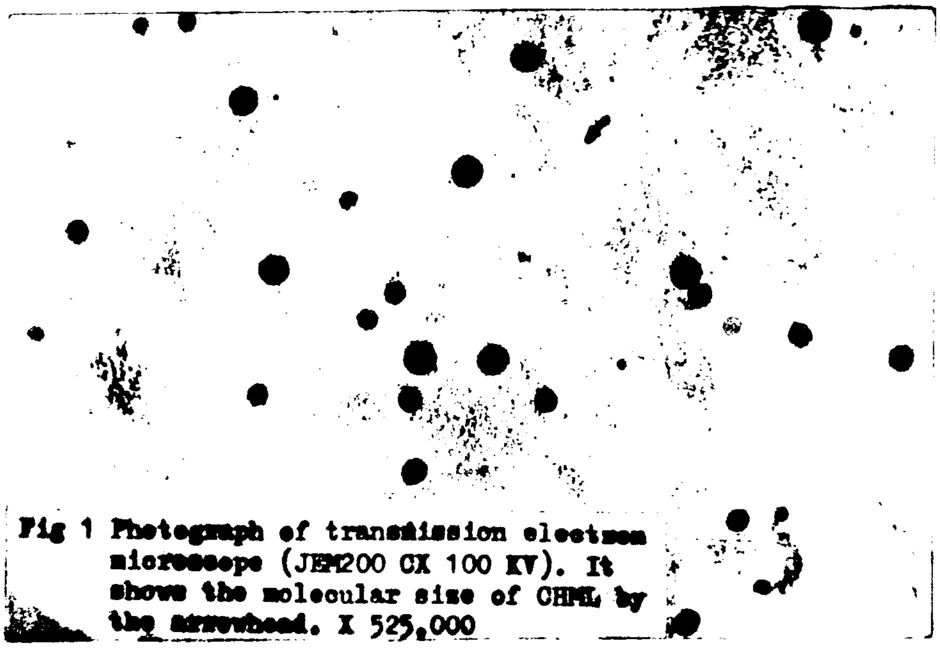

Fig 1 Photograph of transmission electron microscope (JEM200 CX 100 KV). It shows the molecular size of CHML by the arrowhead. X 525,000

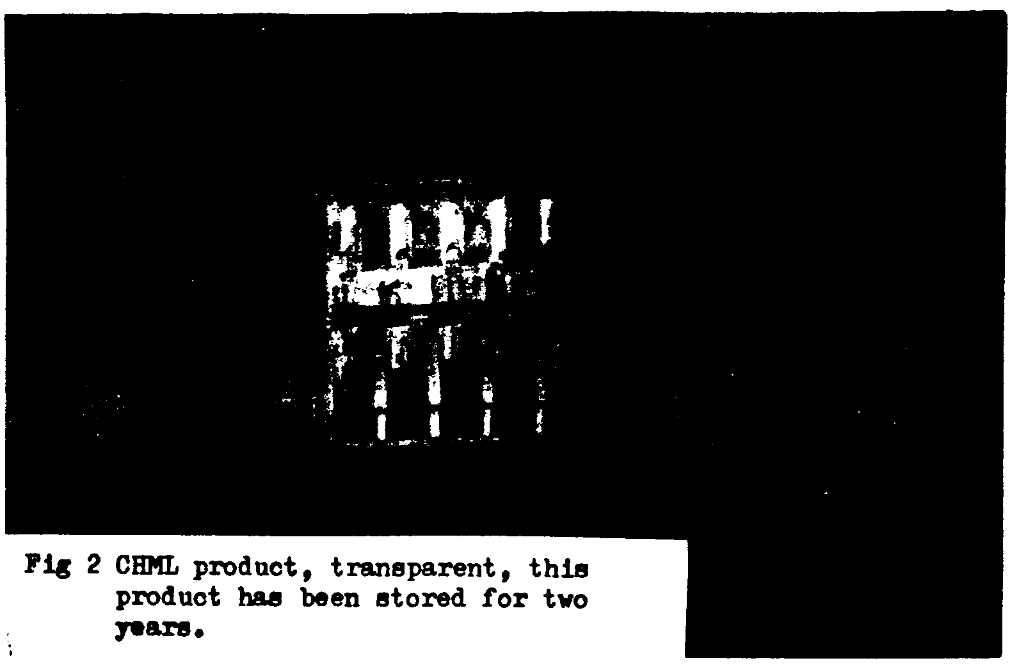

Fig 2 CHML product, transparent, this product has been stored for two years.

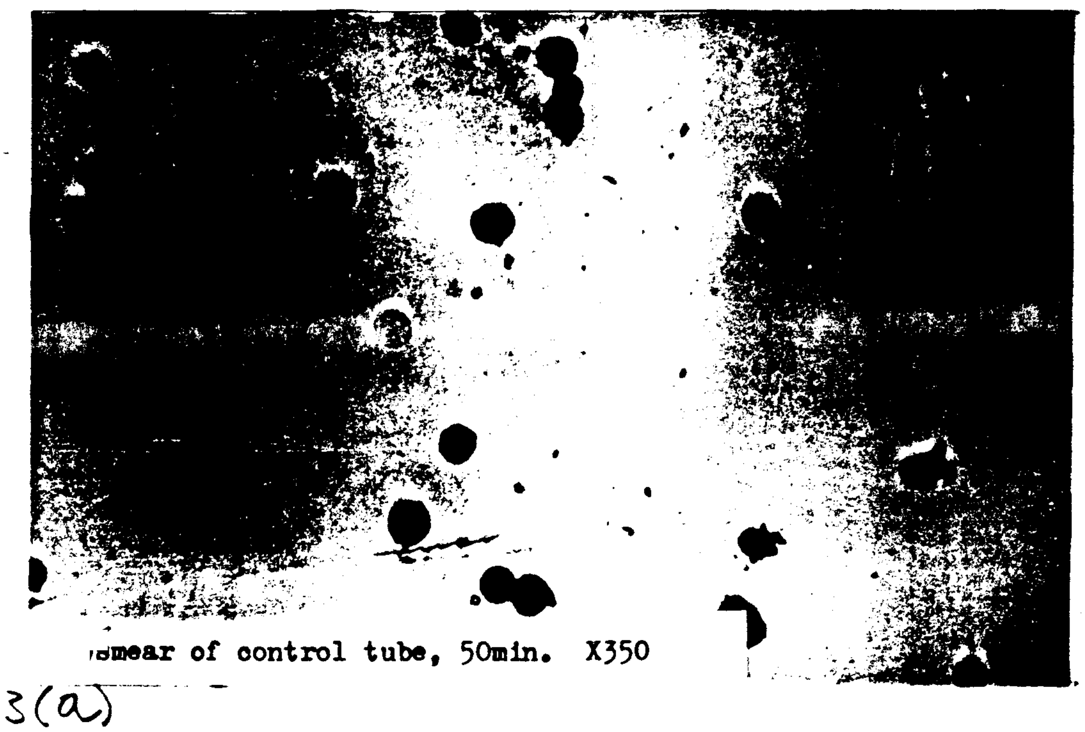

Smear of control tube, 50min. X350

3(a)

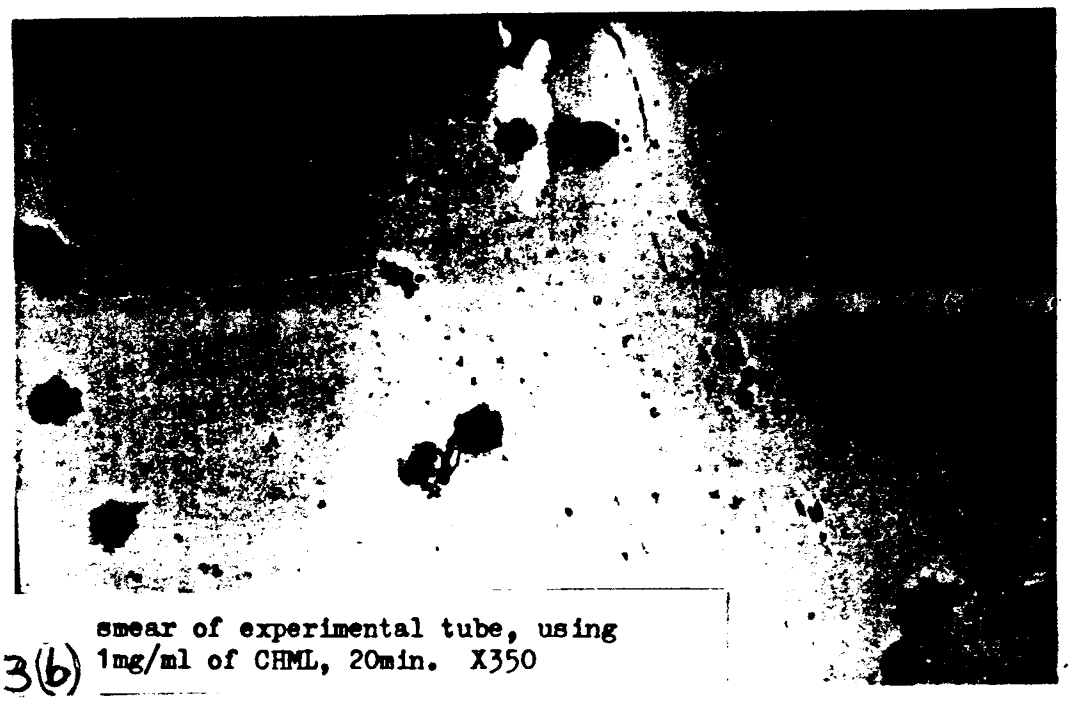

3(b) smear of experimental tube, using 1mg/ml of CHML, 20min. X350

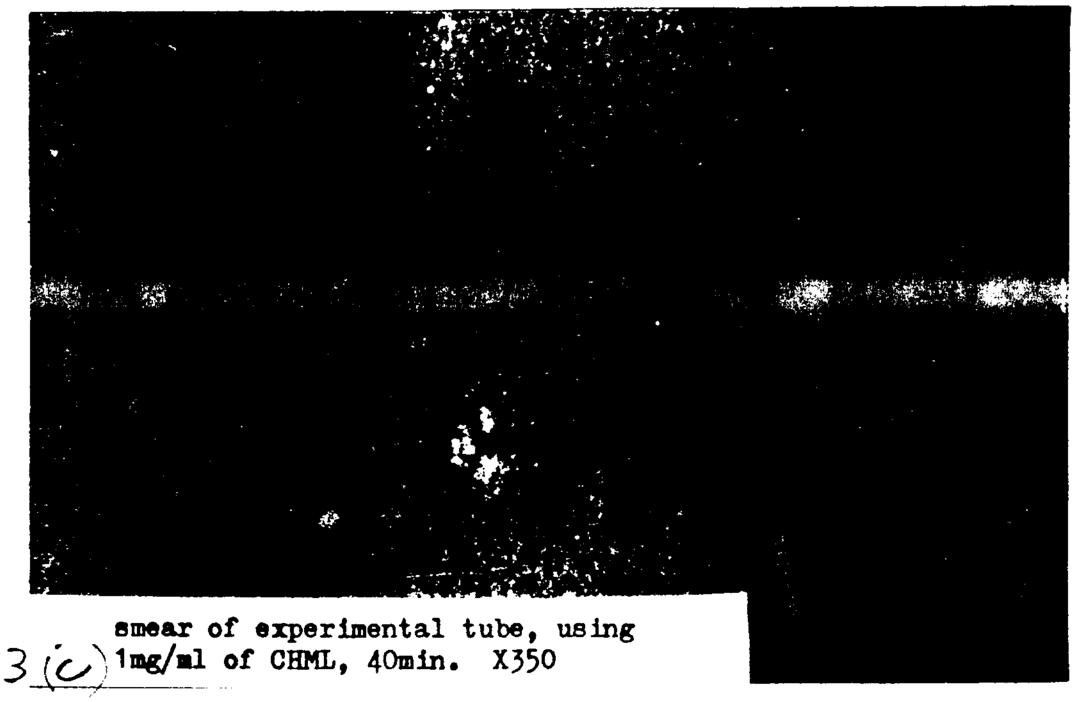

3(c) smear of experimental tube, using 1mg/ml of CHML, 40min. X350

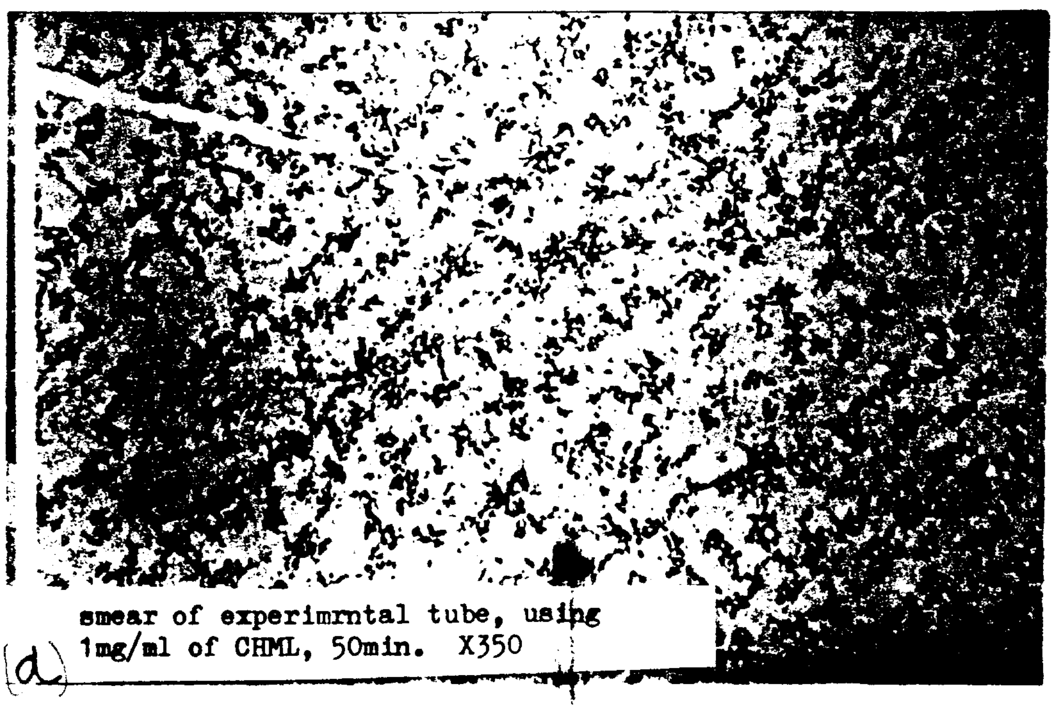

3(d) smear of experimrntal tube, using 1mg/ml of CHML, 50min. X350